# EUROPÄISCHE PATENTSCHRIFT

(11) **EP 0 491 323 B1**
(45) Veröffentlichungstag und Bekanntmachung des Hinweises auf die Patenterteilung: **06.03.1996**
(21) Anmeldenummer: 91121538.2
(22) Anmeldetag: 16.12.1991
(51) Int. Cl.: C07C 255/48, C07C 255/47, C07D 327/10

(54) **Verfahren zur Herstellung von Cyclopropannitrilderivaten**
Process for the preparation of cyclopropanenitrile derivatives
Procédé pour la préparation de dérivés de cyclopropanenitrile

(30) Priorität: 19.12.1990 CH 4029/90
(43) Veröffentlichungstag der Anmeldung: 24.06.1992
(73) Patentinhaber: LONZA AG, CH-3945 Gampel/Wallis (CH)
(72) Erfinder: Hanselmann, Paul, Dr., Visp (Kanton Wallis) (CH)
(74) Vertreter: Weinhold, Peter, Dr.

(56) Entgegenhaltungen:
- EP-A- 0 172 371
- EP-A- 0 332 521
- CHEMICAL ABSTRACTS, vol. 88, no. 7, 13. Februar 1978, Columbus, Ohio, US; abstract no. 50332, S TERATAKE: 'SYNTHESIS OF CYCLOPROPANE DERIVATIVES VIA ORGANOTIN COMPOUNDS' Seite 500 ;
- JOURNAL OF THE AMERICAN CHEMICAL SOCIETY Bd. 79, 1957, WASHINGTON DC Seiten 3467-3469; E R NELSON ET AL: 'SYNTHESIS OF 2,2-DIALKYLCYCLOPROPANE NITRILES'

## Beschreibung

Die Erfindung betrifft ein neues Verfahren zur Herstellung von Cyclopropannitrilderivaten.

2,2-Dimethylcyclopropannitril ist ein wichtiges Zwischenprodukt zur Herstellung von S-(+)-2,2-Dimethylcyclopropancarboxamid, indem dieses zu R,S-2,2-Dimethylcyclopropancarbonsäure hydrolysiert wird und dieses durch Racematspaltung in das optisch reine S-(+)-Enantiomere überführt und nachfolgend via Säurechlorid in das S-(+)-2,2-Dimethylcylopropancarboxamid umgewandelt wird (Lit.: EP 093 511). S-(+)-2,2-Dimethylcyclopropancarboxamid wiederum dient als Ausgangsmaterial zur Herstellung des Dehydropeptidase-Inhibitors Cilastatin, der in der Therapie zusammen mit Penem- bzw. Carbapenem-Antibiotika verabreicht wird, um die Desaktivierung der Antibiotika durch eine renale Dehydropeptidase in der Niere zu verhindern (Lit.: EP 048 301).

Nelson et al, J.Am.Chem.Soc., 79, S.3467-3469 (1957) beschreiben ein Verfahren zur Herstellung von 2,2-Dialkylcyclopropannitrilen, ausgehend von 2,2-Dialkyl-1,3-propandiolen, wobei diese mit p-Toluolsulfonylchlorid in die Ditosylatderivate überführt werden und dann mit Kaliumcyanid zu den 2,2-Dialkylcyclopropannitrilen umgesetzt werden.
Ein grosser Nachteil dieses Verfahrens besteht darin, dass dabei grosse Mengen an Kaliumtosylat als Abfallprodukt zur Entsorgung anfallen.

Die Aufgabe der vorliegenden Erfindung bestand darin, diesen Nachteil auszuschalten und ein ökologisches und wirtschaftlich durchführbares Verfahren zur Herstellung von Cyclopropannitrilderivaten zur Verfügung zu stellen.
Diese Aufgabe wurde gemäss Patentanspruch 1 gelöst.

Das Verfahren zur Herstellung von Cyclopropannitrilderivaten der allgemeinen Formel 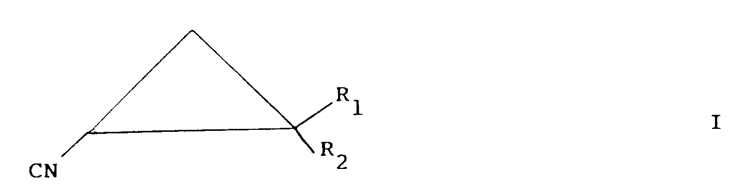 worin R₁ und R₂ gleich oder verschieden sind und ein Wasserstoffatom, eine C₁-C₆-Alkylgruppe, verzweigt oder unverzweigt, eine C₁-C₆-Alkenylgruppe, verzweigt oder unverzweigt, bedeuten, oder worin R₁ und R₂ einen C₄-C₆-Cycloalkylring bedeuten, wird derart durchgeführt, dass man in der ersten Stufe ein Diol der allgemeinen Formel 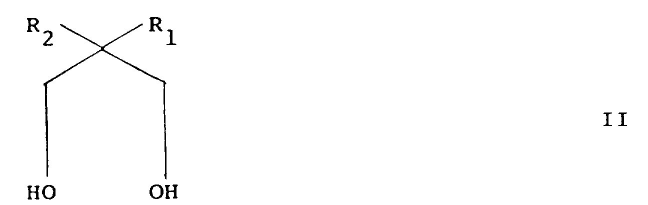 worin R₁ und R₂ die genannte Bedeutung haben, mit Thionylchlorid zu einer Verbindung der allgemeinen Formel 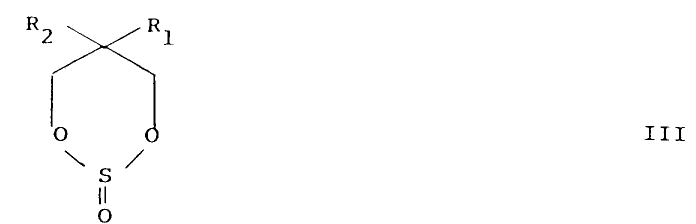 worin R₁ und R₂ die genannte Bedeutung haben, umsetzt, weiter in der zweiten Stufe zu einer Verbindung der allgemeinen Formel 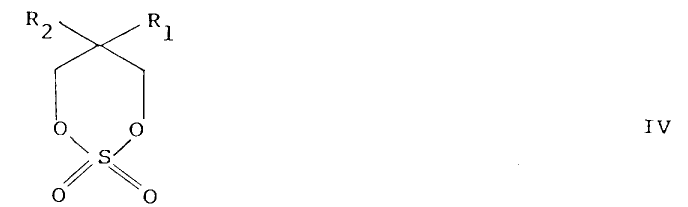 worin R₁ und R₂ die genannte Bedeutung haben, oxidiert, und weiter in der dritten Stufe einem Alkalicyanid bei Temperaturen zwischen 180 °C und 220 °C in das Endprodukt überführt.

Die Edukte, die Diole der allgemeinen Formel II, können beispielsweise durch Alkylierung von Malonsäureesterderivaten und anschliessende Reduktion zum Diol hergestellt werden (Harndem, M.R. und Jarvest, R.L., Tetrahedron Letters, 1985, S.4265).

Zweckmässig wird das Verfahren ausgehend von 2,2-Dimethyl-1,3-propandiol durchgeführt.

Zweckmässig wird die Umsetzung in der ersten Stufe mit 1 bis 3 mol Thionylchlorid, vorzugsweise mit 1 bis 1,5 mol Thionylchlorid, bezogen auf 1 mol Diol der allgemeinen Formel II, durchgeführt.

Die Umsetzung in der ersten Stufe wird zweckmässig bei einer Temperatur von -10 bis 80°C, vorzugsweise von 20 bis 30°C, durchgeführt.

Die Umsetzung in der ersten Stufe kann mit oder ohne Lösungsmittel durchgeführt werden.

Als Lösungsmittel können in der ersten Stufe Alkane,wie Hexan, aromatische Kohlenwasserstoffe, wie Toluol, halogenierte Kohlenwasserstoffe, wie Chloroform, Dichlorethan oder Methylenchlorid, Tetrahydrofuran oder Dioxan angewendet werden. Vorzugsweise werden Toluol, Methylenchlorid oder Dichlorethan eingesetzt.

Die Umsetzung in der ersten Stufe erfolgt üblicherweise in einem Zeitraum von 10 min bis 24 h, vorzugsweise von 1 bis 2 h, wobei das Produkt gemäss Formel III nach fachmännisch üblichen Aufarbeitungsmethoden, wie beispielsweise durch Neutralwaschen oder Destillation, gegebenenfalls isoliert werden kann.

Zweckmässig erfolgt die Umsetzung ohne Isolation des Zwischenproduktes gemäss Formel III.

Beispielsweise kann die Oxidation in der zweiten Stufe entweder mit einem Alkalipermanganat, einer Persäure, einem Alkalichlorat, einem Erdalkalihypochlorit oder mit einem Alkalihypochlorit durchgeführt werden.
Als Alkalipermanganat kann beispielsweise Kaliumpermanganat oder Natriumpermanganat angewendet werden.
Als Persäure kann beispielsweise meta-Chlor-perbenzoesäure angewendet werden.
Als Alkalichlorat kann beispielsweise Natriumchlorat oder Kaliumchlorat angewendet werden.
Als Erdalkalihypochlorit kann beispielsweise Calciumhypochlorit oder Magnesiumhypochlorit dienen.
Als Alkalihypochlorit wird beispielsweise Natriumhypochlorit oder Kaliumhypochlorit angewendet.
Zweckmässig erfolgt die Oxidation in der zweiten Stufe entweder mit einem Alkalipermanganat oder mit einem Alkali- oder Erdalkalihypochlorit.
Das Oxidationsmittel wird zweckmässig in einer Menge von 1 bis 3 mol, vorzugsweise mit 1 bis 2 mol, bezogen auf 1 mol Verbindung der Formel III, angewendet.

Die Oxidation mit einem Alkalipermanganat findet zweckmässig in Gegenwart einer Säure statt.
Als Säuren können beispielsweise Essigsäure oder Mineralsäuren, wie Schwefelsäure oder Salzsäure, angewendet werden. Zweckmässig wird die Säure im Ueberschuss, bezogen auf die Verbindung der Formel III, angewendet.
Vorzugsweise wird die Säure in einer Menge von 2 bis 4 mol, vorzugsweise von 3 mol, bezogen auf 1 mol Verbindung der Formel III, angewendet.

Die Oxidation mit einem Alkali- oder Erdalkalihypochlorit findet zweckmässig in Gegenwart eines Katalysators statt. Als Katalysator kann beispielsweise eine Rutheniumverbindung, eine Eisenverbindung, eine Manganverbindung, eine Wolframverbindung oder eine Chromverbindung angewendet werden.

Zweckmässig dient als Katalysator ein Rutheniumtrihalogenid, wie beispielsweise Rutheniumtrichlorid, Rutheniumtribromid oder Rutheniumtrijodid. Als Katalysator kann auch eine Ruthenium-Sauerstoffverbindung, wie beispielsweise Rutheniumdioxid oder Rutheniumtetroxid, angewendet werden. Vorzugsweise wird Rutheniumtrichlorid oder Rutheniumdioxid angewendet.

Zweckmässig wird der Katalysator in der zweiten Stufe in einer Menge von 10 bis 0,00001 mol% angewendet, vorzugsweise von 0,1 bis 0,001 mol%.

Als Lösungsmittel können für die Oxidation halogenierte Kohlenwasserstoffe, wie beispielsweise Methylenchlorid oder 1,2-Dichlorethan, Ester,wie beispielsweise Essigsäureethylester, Ether wie beispielsweise Diethylether, Acetonitril oder aromatische Kohlenwasserstoffe,wie beispielsweise Toluol,angewendet werden. Vorzugsweise wird Essigsäureethylester, Methylenchlorid oder Toluol angewendet.

Die Oxidation in der zweiten Stufe erfolgt zweckmässig bei einer Temperatur von 0 bis 80°C, vorzugsweise von 20 bis 30°C.

Nach einer üblichen Reaktionszeit von 0,5 bis 24 h, vorzugsweise von 3 bis 6 h, kann das Produkt gemäss Formel IV nach fachmännisch üblichen Aufarbeitungsmethoden, beispielsweise durch Kristallisation, gegebenenfalls isoliert werden. Vorzugsweise wird das Produkt gemäss Formel IV isoliert.

Die Umsetzung in der dritten Stufe erfolgt mit einem Alkalicyanid.
Als Alkalicyanid findet beispielsweise Natriumcyanid oder Kaliumcyanid Anwendung.

Das Alkalicyanid wird zweckmässig in einer Menge von 1 bis 3 mol, vorzugsweise 1 bis 2 mol, bezogen auf 1 mol Verbindung der Formel IV, angewendet.

Zweckmässig erfolgt die Umsetzung in der dritten Stufe mit einem Alkalicyanid in Gegenwart einer Base.
Als Base kann beispielsweise ein Alkalicarbonat oder ein Alkalihydrogencarbonat angewendet werden.
Als Alkalicarbonat kann beispielsweise Natriumcarbonat oder Kaliumcarbonat, als Alkalihydrogencarbonat Natriumhydrogencarbonat oder Kaliumhydrogencarbonat dienen.
Die Base wird zweckmässig in einer Menge von 1 bis 2 mol, bezogen auf 1 mol Alkalicyanid, angewendet.

Zweckmässig wird die Umsetzung in der dritten Stufe in einem polaren Lösungsmittel, wie beispielsweise in Glycolderivaten, wie Ethylenglycol, Diethylenglycol, Ethylenglycolmonomethylether, Ethylenglycolmonoethylether, Diethylenglycolmonomethylether oder Diethylenglycolmonoethylether, durchgeführt. Vorzugsweise wird Ethylenglycol angewendet.

Die Umsetzung in der dritten Stufe kann auch in Dimethylsulfoxid als Lösungsmittel unter Druck durchgeführt werden.

Zweckmässig wird die Umsetzung in der dritten Stufe bei einer Temperatur von 180 bis 220°C durchgeführt.

Nach einer üblichen Reaktionszeit von 1 bis 24 h, vorzugsweise 2 bis 5 h, kann das Endprodukt gemäss Formel I nach fachmännisch üblichen Aufarbeitungsmethoden, z.B. durch Destillation, isoliert werden.

Vorzugsweise wird das erfindungsgemässe Verfahren als Eintopfverfahren durchgeführt, wobei gegebenenfalls das Zwischenprodukt gemäss Formel IV isoliert wird.

Die Cyclopropannitrilderivate der allgemeinen Formel 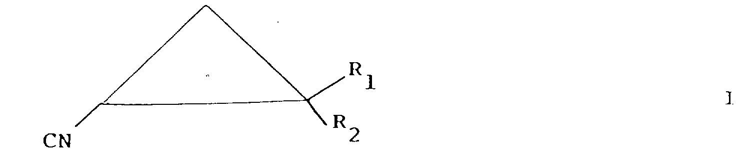 worin R₁ und R₂ einen C₄-C₆-Cycloalkylring bedeuten, für die jedoch kein Schutz begehrt wird, können zur entsprechenden C₄-C₆-Cycloalkylpropancarbonsäure hydrolysiert werden. Ein bevorzugter Vertreter dieser Derivate ist 1-Cyanospiro[4,2]heptan, worin R₁ und R₂ einen C₅-Cycloalkylring bedeuten.

Erfindungsgemäss wird ein ökologisches und insbesondere ein billiges, wirtschaftliches Verfahren zur Herstellung von Cyclopropannitrilderivaten zur Verfügung gestellt, wobei die dabei anfallenden Salze, wie beispielsweise Natrium- bzw. Kaliumsulfat, im Gegensatz zu dem von Nelson et al, J.Am.Chem. Soc., 79, S.3467-3469 (1957) beschriebenen Verfahren, wesentlich leichter zu entsorgen sind als Kaliumtosylat.

### Beispiel 1

### Herstellung von 5,5-Dimethyl-1,3,2-dioxathian-2-oxid

In einem 500 ml-Dreihalskolben wurden 25,36 g (0,24 mol) 2,2-Dimethylpropan-1,3-diol bei Raumtemperatur in 150 ml Methylenchlorid aufgeschlämmt. Während 30 min wurden 37,36 g (0,31 mol) Thionylchlorid zugetropft.

Nach Ende der Zugabe wurde für 1,5 h die Reaktionslösung am Rückfluss gehalten und dann auf Raumtemperatur abgekühlt. Zur Reaktion wurden 100 ml Wasser gegeben. Die wässrige Phase wurde verworfen, die organische Phase wurde 3 mal mit gesättigter NaHCO₃-Lösung (100 ml) extrahiert, anschliessend mit Na₂SO₄ getrocknet und am Rotationsverdampfer eingeengt. Der Rückstand wurde bei 24 mbar und 78°C destilliert.

Das Produkt war eine nach GC reine farblose Flüssigkeit und wog 23,4 g, was einer Ausbeute von 92% entsprach.

### Beispiel 2

### Herstellung von 5,5-Dimethyl-1, 3, 2-dioxathian-2, 2-dioxid

### a) mit Kaliumpermanganat

In einem 250 ml-Dreihalskolben wurden 5 g (0,03 mol) 5,5-Dimethyl-1,3,2-dioxathian-2-oxid in 100 ml Methylenchlorid vorgelegt. Dazu wurden 100 ml Wasser und 8,22 g (0,08 mol) konz. Schwefelsäure zugetropft. Dann wurden langsam 5,22 g (0,03 mol) Kaliumpermanganat portionenweise zugegeben. Die Reaktionssuspension wurde über Nacht bei 30°C gerührt, dann wurden die Phasen filtriert. Die organische Phase wurde 3 mal mit gesättigter NaHCO₃-Lösung (100 ml) extrahiert, anschliessend getrocknet und eingeengt.
Aus dem Rückstand wurden die Kristalle abfiltriert und mit Hexan gewaschen.
Das Produkt hatte einen Smp. von 71-74°C und wo 2,92 g, was einer Ausbeute von 58% entsprach.

### b) mit Natriumhypochlorit

In einem 250 ml-Dreihalskolben wurden 10,0 g (0,064 mol) 5,5-Dimethyl-1,3,2-dioxathian-2-oxid bei 20°C in 100 ml Methylenchlorid und 50 ml Wasser vorgelegt. Als Katalysator wurden 0,02 g 90%iges Rutheniumtrichlorid zugegeben. Unter Rühren wurden während 20 min 152,45 g (0,0727 mol) Natriumhypochlorit in Wasser zugesetzt, wobei die Temperatur auf 30°C anstieg.

Die Reaktion wurde noch 5 h gerührt und mit 10 Tropfen Isopropanol versetzt, wobei die gelbe Farbe verschwand und sich ein schwarzer Niederschlag bildete.
Die Wasserphase wurde 2 mal mit Methylenchlorid (50 ml) extrahiert und die vereinigten organischen Phasen mit NaHCO₃- Lösung (100 ml) extrahiert, mit Na₂SO₄ getrocknet und eingeengt.
Das weisse kristalline Produkt wurde über Nacht bei Raumtemperatur bei einem Druck von 15 mbar getrocknet und wog 10,65 g, was einer Ausbeute von 99% entsprach, bezogen auf eingesetztes 5,5-Dimethyl-1,3,2-dioxathian-2-oxid.

### Beispiel 3

### Herstellung von 2, 2-Dimethylcyclopropannitril

### a) mit Kaliumcyanid

In einem 200 ml-Zweihalskolben wurden bei Raumtemperatur 78 g Ethylenglycol, 5 g (28,6 mmol) 5,5-Dimethyl-1,3,2-dioxathian-2, 2-dioxid mit 4,74 g (71,3 mmol) Kaliumcyanid vorgelegt. Die Reaktionssuspension wurde auf 200°C erwärmt und so lange bei dieser Temperatur gehalten, bis die Hälfte des Ethylenglycols abdestilliert war.
Das Destillat (Ethylenglycol und Produkt) wurde 3 mal mit Pentan (50 ml) extrahiert und die vereinigten Pentanphasen bei Normaldruck eingeengt.
Der Rückstand betrug 2,0 g und enthielt nach GC 2,2-Dimethylcyclopropannitril.
Die Ausbeute betrug somit 67%, bezogen auf 5,5-Dimethyl-1,3,2-dioxathian-2,2-dioxid.

Zur endgültigen Reinigung wurde das Produkt am Wasserstrahlvakuum destilliert (Sdp. ~ 45°C, 30 mbar).

### b) mit Natriumcyanid

In einem 1000 ml-Zweihalskolben wurden in 750 ml (834,8 g, 13,449 mol) Ethylenglykol 100 g (0,590 mol) 5,5-Dimethyl-1,3,2-dioxathian-2,2-dioxid und 75 g (1,504 mol) Natriumcyanid vorgelegt.
Die Reaktionssuspension wurde im Laufe von 1,5 h auf eine Temperatur von 220°C gebracht, wobei ein Gemisch aus Ethylenglykol und Produkt abdestilliert wurde.
Es wurde solange destilliert, bis die Hälfte des Ethylenglykols (400 ml) in der Vorlage war.
Der Inhalt der Vorlage wurde erneut bei 30°C und 150 mbar destilliert.
Das Produkt wog 61,3 g und enthielt ca. 10% Wasser nach GC. Somit beträgt die Ausbeute etwa 90%, bezogen auf 5,5-Dimethyl--1,3,2-dioxathian-2,2-dioxid.

### Beispiel 4

### Herstellung von 5,5-Dimethyl-1,3,2-dioxathian-2,2-dioxid Eintopfverfahren:

### a) mit Natriumhypochlorit

In einem 1,5-l-Dreihalskolben wurden bei Raumtemperatur 100,1 g (0,942 mol) 98%iges 2,2-Dimethyl-1,3-propandiol vorgelegt und mit 113,6 g (0,952 mol) 99,5%igem Thionylchlorid unter Rühren versetzt. Es trat eine starke Gasentwicklung ein, dabei sank die Temperatur von Raumtemperatur auf 5°C,und es entstand eine Lösung. Nach 1 h wurden 300 ml Essigsäureethylester zugegeben und die Essigsäureethylesterlösung 2 mal mit gesättigter NaHCO₃-Lösung (150 ml) extrahiert. Die wässrige Phase wurde verworfen.
Der organischen Phase wurden 0,02 g (0,0001 mol) 90%iges Rutheniumtrichlorid zugesetzt, und dann wurden während 1 h 1078 g (1,5 mol) einer 10,5%igen wässrigen Natriumhypochloritlösung zugetropft. Dabei stieg die Reaktionstemperatur bis an den Siedepunkt des Essigesters an.
Nach Ende der Zugabe wurde die Reaktion über Nacht stehen gelassen und dann mit 5 ml Isopropanol versetzt. Der schwarze Katalysator wurde abfiltriert und die Phasen getrennt.
Die organische Phase wurde mit Na₂SO₄ getrocknet und am Rotationsverdampfer bei 35°C und 90 mbar eingeengt. Nach dem Entfernen von 50% des Essigsäureethylesters begann das Produkt auszufallen.
Die entstandene Suspension wurde ins Eisbad gestellt und die ausgefallenen weissen Kristalle abfiltriert, dann bei 50°C und 50 mbar über Nacht im Trockenschrank getrocknet.
Das Produkt wog 90,9 g und hatte einen Smp. von 80°C.
Die Mutterlauge wurde total eingeengt und ergab nach Trocknung nochmals 32,2 g mit einem Smp. von 78°C.
Die Ausbeute an reinem Produkt betrug somit 78%, bezogen auf eingesetztes 2,2-Dimethyl-1,3-propandiol.

### b) mit Calciumhypochlorit

In einem 500 ml-Dreihalskolben wurden 31,0 g (0,289 mol) 100%-iges 2,2-Dimethyl-1,3-propandiol bei Raumtemperatur in 185 ml Toluol aufgeschlämmt. Danach wurden 34,9 g (0,294 mol) 99,5%-iges Thionylchlorid zugetropft, wobei eine Gasentwicklung eintrat. Die Reaktion wurde zuerst durch Erwärmen und dann Kühlen zwischen 20 bis 30°C gehalten. Nach Ende der Thionylchloridzugabe hatte sich alles Propandiol aufgelöst. Die Toluolphase wurde mit 50 ml gesättigter Na₂CO₃-Lösung gewaschen.
Zur organischen Phase wurden 0,006 g (3,8·10⁻⁵ mol) 90%-iges Rutheniumtrichlorid und 200 ml Wasser gegeben. Unter kräftigem Rühren wurden nun 43,7 g (0,206 mol) 67,5%-iges Calciumhypochlorit eingestreut. Die Temperatur der Reaktion wurde durch Kühlen bei 25 bis 30°C gehalten. Die entstandene weisse Suspension/Emulsion wurde mit 5,7 ml (6,7 g, 0,067 mol) 37%-iger HCl versetzt, wobei sich zwei klare Phasen bildeten, welche getrennt wurden. Die Toluolphase wurde mit Na₂SO₄ getrocknet und am Rotationsverdampfer zur Trockene eingeengt. Der Rückstand wog 40,6 g und bestand zu 98,1 Gew.% aus dem gewünschten Produkt. Die Ausbeute betrug 81%,bezogen auf eingesetztes 2,2-Dimethyl-1,3-propandiol.

### Beispiel 5

### Herstellung von 2,2-Dimethylcyclopropannitril mit Natriumcyanid und Natriumcarbonat als Base

In einem 1000ml-Zweihalskolben wurden bei Raumtemperatur 500 ml (556 g, 8,966 mol) Ethylenglykol mit 49,9 g (0,300 mol) 5,5-Dimethyl-1,3,2-dioxathian-2,2-dioxid, 15 g (0,306 mol) Natriumcyanid und 31,8 g (0,300 mol) Natriumcarbonat vorgelegt.
Im Laufe von 1,5 h wurde die Reaktionssuspension auf 220°C erwärmt, wobei ein Gemisch aus Produkt und Ethylenglykol abdestilliert wurde.
Die Destillation wurde solange fortgesetzt, bis kein Ethylenglykol mehr abdestilliert wurde.
Die Vorlage (504 g) wurde erneut bei 90°C und 150 mbar destilliert, wobei 21,78 g Produkt erhalten wurden. Somit betrug die Ausbeute 67%, bezogen auf das eingesetzte 5,5-Dimethyl-1,3,2-dioxathian-2,2-dioxid.

### Beispiel 6

### Herstellung von 1-Cyanospiro[4,2]heptan

Bei Raumtemperatur wurden in 40 ml Ethylenglykol 1,27 g (0,026 mol) Natriumcyanid und 5,55 g (0,026 mol) 3,5,4-Dioxathiaspiro[4,5]decan-4,4-dioxid aufgeschlämmt und für 15 min auf 150°C erwärmt. Dann liess man die Reaktionsmischung auf 80°C abkühlen und setzte 5,5 g (0,052 mol) Na₂CO₃ (wasserfrei) zu. Über eine einfache Destillationsbrücke wurden 22,3 g Ethylenglykol abdestilliert, bei 225°C Badtemperatur. Das Destillat enthielt etwa 1% der Spiroverbindung, welche anhand ihres MS-Spektrums insbesondere ihres typischen (M-1)⁺ von 120 identifiziert wurde.

## Patentansprüche

1. Verfahren zur Herstellung von Cyclopropannitrilderivaten der allgemeinen Formel 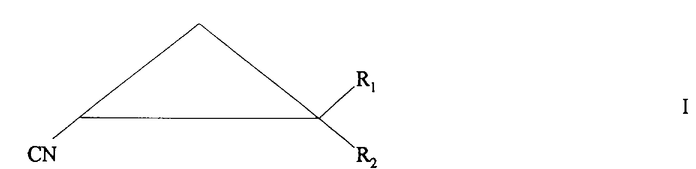 worin R₁ und R₂ gleich oder verschieden sind und ein Wasserstoffatom, eine C₁-C₆-Alkylgruppe, verzweigt oder unverzweigt, eine C₁-C₆-Alkenylgruppe, verzweigt oder unverzweigt, bedeuten, oder worin R₁ und R₂ einen C₄-C₆-Cycloalkylring bedeuten, dadurch gekennzeichnet, dass man in der ersten Stufe ein Diol der allgemeinen Formel 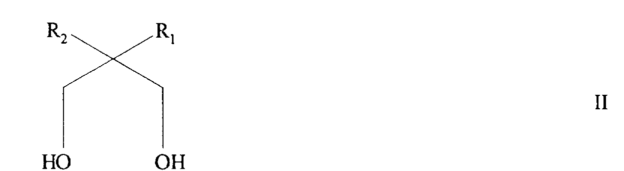 worin R₁ und R₂ die oben genannte Bedeutung haben, mit Thionylchlorid zu einer Verbindung der allgemeinen Formel 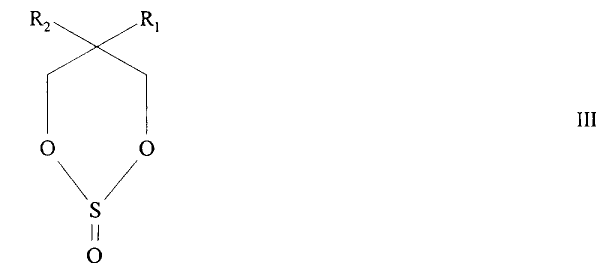 worin R₁ und R₂ die oben genannte Bedeutung haben, umsetzt, weiter in der zweiten Stufe zu einer Verbindung der allgemeinen Formel 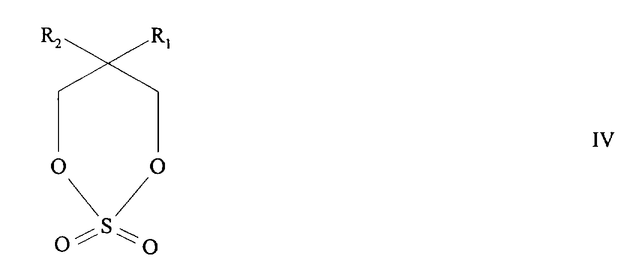 worin R₁ und R₂ die oben genannte Bedeutung haben, oxidiert und weiter in der dritten Stufe mit einem Alkalicyanid bei Temperaturen zwischen 180 °C und 220 °C in das Endprodukt überführt.

2. Verfahren nach Patentanspruch 1, dadurch gekennzeichnet, dass man die Umsetzung in der ersten Stufe mit 2,2-Dimethyl-1, 3-propandiol durchführt.

3. Verfahren nach mindestens einem der Patentansprüche 1 und 2, dadurch gekennzeichnet, dass man die Umsetzung in der ersten Stufe mit 1 bis 3 mol Thionylchlorid, bezogen auf 1 mol Diol, bei einer Temperatur von -10 bis 80°C durchführt.

4. Verfahren nach Patentanspruch 1, dadurch gekennzeichnet, dass man die Oxidation in der zweiten Stufe entweder mit einem Alkalipermanganat oder mit einem Alkali- oder Erdalkalihypochlorit bei einer Temperatur von 0 bis 80°C durchführt.

5. Verfahren nach Patentanspruch 4, dadurch gekennzeichnet, dass man die Umsetzung mit einem Alkali- oder Erdalkalihypochlorit in Gegenwart eines Katalysators durchführt.

6. Verfahren nach Patentanspruch 5, dadurch gekennzeichnet, dass die Umsetzung mit dem Alkalicyanid in Gegenwart einer Base erfolgt.

7. Verfahren nach mindestens einem der Patentansprüche 5 und 6, dadurch gekennzeichnet, dass als Base ein Alkalicarbonat oder ein Alkalihydrogencarbonat verwendet wird.

8. Verfahren nach einem oder mehreren der Patentansprüche 1 bis 7, dadurch gekennzeichnet, dass man das Verfahren ohne Isolierung der Zwischenstufen durchführt.

## Claims

1. Process for the preparation of cyclopropanenitrile derivatives of general formula 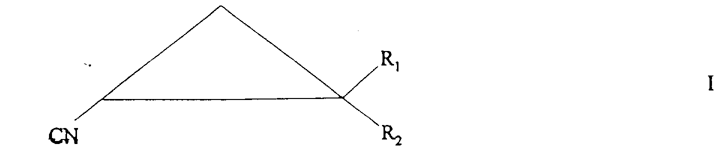 wherein R₁ and R₂ are the same or different and represent a hydrogen atom, a C₁-C₆-alkyl group, branched or straight, a C₁-C₆-alkenyl group, branched or straight, or wherein R₁ and R₂ represent a C₄-C₆-cycloalkyl ring, characterized in that in the first stage a diol of general formula 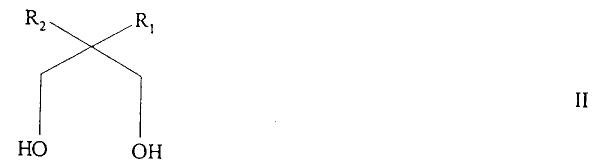 wherein R₁ and R₂ have the above mentioned meaning, is reacted with thionyl chloride to give a compound of general formula 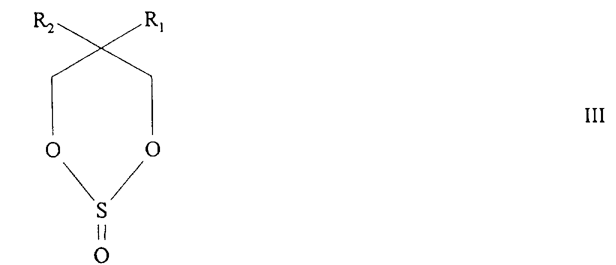 wherein R₁ and R₂ have the above mentioned meaning, in the second stage is further oxidized to a compound of general formula 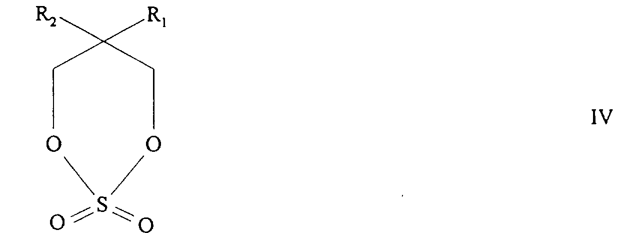 wherein R₁ and R₂ have the above mentioned meaning, and in the third stage is further transferred to the final product with an alkali cyanide at temperatures of from 180 °C to 220 °C.

2. Process according to claim 1, characterized in that the reaction in the first stage is carried out using 2,2-dimethyl-1, 3-propanediol.

3. Process according to at least one of claims 1 and 2, characterized in that the reaction in the first stage is carried out using 1 to 3 moles of thionyl chloride with respect to 1 mol of diol at a temperature of from -10 °C to 80 °C.

4. Process according to claim 1, characterized in that the oxidation in the second stage is carried out using either an alkali permanganate or an alkali or alkaline earth hypochlorite at a temperature of from 0 to 80 °C.

5. Process according to claim 4, characterized in that the reaction is carried out with an alkali or alkaline earth hypochlorite in the presence of a catalyst.

6. Process according to claim 5, characterized in that the reaction with the alkali cyanide is carried out in the presence of a base.

7. Process according to at least one of claims 5 and 6, characterized in that an alkali carbonate or an alkali hydrogencarbonate is used as the base.

8. Process according to one or more of claims 1 to 7, characterized in that the process is carried out without isolation of the intermediate products.

## Revendications

1. Procédé pour la préparation de dérivés de cyclopropanenitrile de formule générale 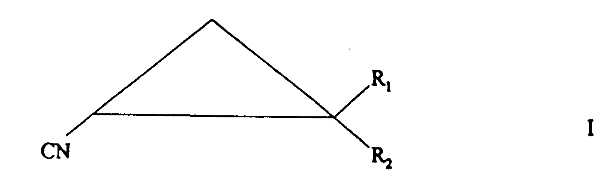 dans laquelle R₁ et R₂ sont identiques ou différents et signifient un atome hydrogène, un groupe alkyle C₁-C₆, ramifié ou non ramifié, un groupe alcényle C₁-C₆, ramifié ou non ramifié, ou dans laquelle R₁ et R₂ signifient un sommet cycloalkyle C₄-C₆, caractérisé en ce que dans la première étape, on transforme un diol de formule générale 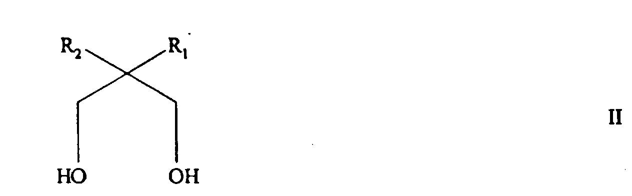 dans laquelle R₁ et R₂ ont la signification citée, avec un chlorure de thionyle on transforme en un composé de la formule générale 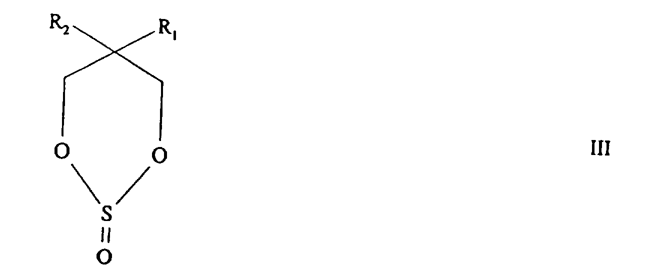 dans laquelle R₁ et R₂ ont la signification précitée, on oxyde dans la deuxième étape en un composé de la formule générale 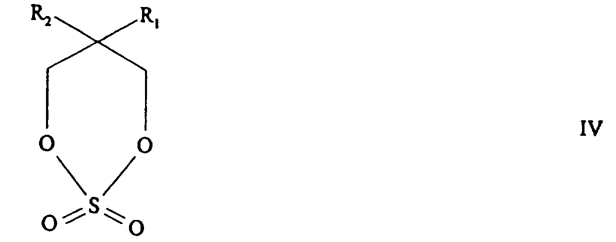 dans laquelle R₁ et R₂ ont la signification précitée et dans la troisième étape avec un cyanure alcalin, on transforme à des températures entre 180 et 220°C en le produit final.

2. Procédé selon la revendication 1, caractérisé en ce que dans la première étape, la réaction est conduite avec le 2,2-diméthyl-1,3-propandiol.

3. Procédé selon au moins l'une des revendications 1 et 2, caractérisé en ce que dans la première étape, on conduit la réaction avec 1 à 3 moles de chlorure de thionyle rapportés à 1 mole de diol à une température de -10 à 80°C.

4. Procédé selon la revendication 1, caractérisé en ce que l'on conduit l'oxydation dans la deuxième étape soit avec un permanganate alcalin soit avec un hypochlorite alcalin ou alcalino-terreux à une température de 0 à 80°C.

5. Procédé selon la revendication 4, caractérisé en ce que la réaction est conduite avec un hypochlorite alcalin ou alcalino-terreux en présence d'un catalyseur.

6. Procédé selon la revendication 5, caractérisé en ce que la réaction s'effectue avec le cyanure alcalin en présence d'une base.

7. Procédé selon au moins l'une des revendications 5 et 6, caractérisé en ce que comme base, on utilise un carbonate alcalin ou un hydrogénocarbonate alcalin.

8. Procédé selon une ou plusieurs des revendications 1 à 7, caractérisé en ce que l'on conduit le procédé sans isolation des étapes intermédiaires.
